# EUROPEAN PATENT SPECIFICATION

(11) **EP 2 009 432 B1**
(45) Date of publication and mention of the grant of the patent: **11.08.2010**
(21) Application number: 08157462.6
(22) Date of filing: 03.06.2008
(51) Int. Cl.: G01N 27/12, G01N 27/04, G01N 27/22

(54) **Package tampering and humidty sensor and the use thereof**
Zeit- und Feuchtesensor und dessen Verwendung
Capteur de temps et d'humidité et son utilisation

(30) Priority: 27.06.2007 FI 20075494
(43) Date of publication of application: 31.12.2008
(73) Proprietor: Valtion Teknillinen Tutkimuskeskus, 02150 Espoo (FI)
(72) Inventor: Vilkman, Marja, FI-00670, Helsinki (FI); Mäkelä, Tapio, FI-00740, Helsinki (FI); Lehtinen, Kaisa, FI-01520, Vantaa (FI)
(74) Representative: Hakkila, Maini Annika

(56) References cited:
- EP-A- 1 139 092
- WO-A-02/058080
- US-A- 4 298 855
- US-A- 5 310 507

## Description

### Field of the Invention

The invention is related to a package tampering and humidity sensor, incorporated in a package or packaging material of goods, such as electronics, food products and perishable products. The package tampering and humidity sensor, incorporated in a package or packaging material, comprises a layered structure, which is based on a conductive polymer, and it indicates whether the package or packaging material has been exposed to a humid environment after activation of the sensor.

### State of the Art

Typically perishable products like medical supplies, drugs, cosmetics, chemicals, foodstuffs and electronics are supplied in tightly sealed packages, which provide containment, and optionally specific atmosphere for the prevention of contamination and tampering. It is essential to ensure the shelf life and quality of the product, as specified by the supplier.

Various temperature-sensitive indicators useful for monitoring the storage of highly perishable products such as food products, drugs, biological materials, chemicals, coating compositions, adhesives, cosmetics, food additives, materials for photography, vaccines and the like are known. It is important for consumers, retail trade wholesalers, and producers of products to readily notice any changes or problems in the quality of the product with for instance electrical or optical means.

Temperature detectors used at present mainly indicate the maximum or minimum storage temperature of the product, and they are normally attached as self-adhesive labels on the product packages, such as food packages.

An electrochemical sensor adapted to detect oxygen or other analyte, such as carbon dioxide, hydrogen sulfide, ethanol, alkyl sulfide, ketones, aldehydes, esters, ammonia or amines, in the interior of a package, and adapted to generate a signal on the exterior of the package is disclosed in WO 9602438. Particularly, a sensor element is attached to an impermeable packaging material at a preformed hole, comprising a substrate, such as polymeric material, laminate, metallized film, metal foil or waxed paper, a sensing layer, which may be an electrolyte or conductor connected to the sensing layer and comprising a conducting polymer and an electrical contact communicating with the exterior of the package.

W0 2002058080 discloses a layered structure formed of a conductive polymer on a substrate material. The substrate material has the property of catalyzing changes in the layered structure when it experiences changes in the surrounding environment, like becoming exposed to humidity. The substrate material causes dedoping reaction in the conductive polymer. Also a sensor is provided comprising an electric device short-circuited by the conductive polymer layer of the structure.

A moisture sensor is presented in JP 1124755, comprising a first and a second electrode on a substrate consisting of quartz glass. A moisture sensitive film of polyaniline etc is provided on the electrodes and spacings between them.

A humidity sensor is disclosed in EP 1 139 092, said humidity sensor including a pair of interdigital electrodes disposed on an insulating substrate and defining a gap there between, an undercoat layer of silane compound lying on the gap-defining electrodes and a substrate, and a humidity sensitive thin film lying thereon. Said humidity sensitive thin film is formed of a cross-linked product of a conductive polymer physically bound to the silane compound.

A method for manufacturing a conductive polymer selective species sensor for irreversibly detecting the exposure of the sensing element to a first dopant or a first compensating agent in the presence of a second dopant or a second compensating agent is provided in US 5,310,507.

US 4,298,855 describes a humidity sensor comprising a conductive polymer film containing particles of carbon to form the conductive (or resistive) portions in the sensor.

Conductive polymers are materials, which are made conductive by doping with a suitable substance. In the doping process both electron acceptors and donors react with the polymer structure resulting in highly conducting derivatives. These reactions are called 'doping' in analogy to semiconductors, though they are closer to solid-state chemical reactions. Another method for generating electrical conductivity in polymers is blending where conductive polymer or other conductive material, like metal, and insulating material are mixed. Electrical properties of conductive polymers can be changed over the full range of conductivity from insulators to metallic conductors.

An example of a conductive polymer is polyaniline (PANI), which is a synthetic organic polymer obtained by chemical or electrochemical oxidative polymerization of aniline. Polyaniline is a semiconductor in its emeraldine base form but it can be made conducting if it is protonated or doped with counter-ions. As common conductivity behavior of polyaniline complexes in humid conditions, increase of conductivity is noticed according to Nechtschein, M., Santier, C., Travers, J.P., Chroboczek, J., Alix, A., Ripert, M., Synthetic Metals 18 (1987) 311-316, Javadi, H.H.S., Angelopoulos, M., MacDiarmid, A.G., Epstein, A.J., Synthetic Metals 26 (1988) 1-8, Lubentsov, B.Z., Timofeeva, O.N., Khidekel, K.L., Synthetic Metals 45 (1991) 235-240.

US 5,783,111 discloses compositions comprising electrically conducting polyaniline and particularly substituted aromatic compounds that simultaneously form hydrogen bonds and ring-ring interactions with the NH-groups and six-membered rings of conducting polyanilines. Conducting polyaniline compositions show drastically enhanced processability and their blends with insulating or semiconducting materials exhibit significantly improved electrical properties.

None of the prior art references disclose a sensor utilizing irreversible reactions of conductive polymers.

Based on what is said above, there is an evident need for a package tampering and humidity or moisture sensitive sensor, which may easily be incorporated into the particular packing material of a product, thus avoiding the steps of attachment of labels, and provides irreversible reactions of conductive polymers.

### Object of the Invention

An object of the invention is to provide a package tampering and humidity sensitive sensor that may easily be incorporated into the packing material of a product.

A further object of the invention is a package tampering and humidity sensitive sensor, which provides irreversible reactions of conductive polymers.

A further object of the invention is a method for the manufacture of the package tampering and humidity sensitive sensor, which is easy to incorporate into packing materials.

A still further object of the invention is the use of the package tampering and humidity sensitive sensor in packages and packaging material of foodstuff, electronics and perishable products, for monitoring whether the package or packaging material has been exposed to a humid or moist environment after activation of the sensor.

A still further object of the invention is a package and packaging material for foodstuff, electronics and perishable products comprising the package tampering and humidity sensitive sensor.

Characteristic features of the package tampering and humidity sensitive sensor, of the method for the manufacture thereof, of the use thereof and packages and packaging materials comprising the sensor are provided in the claims.

The package tampering and humidity sensitive sensor means here that the sensor is sensitive to humidity, moisture and any forms of water vapor.

### Summary of the Invention

The present invention relates to a package tampering and humidity sensitive sensor that may easily be incorporated into packages or packing materials of products, particularly into packaging materials for foodstuff, electronics and perishable products, and which provides irreversible reactions of conductive polymers.

The invention relates also to a method for the manufacture of the package tampering and humidity sensitive sensor that may easily be incorporated into the packing material of a product. The invention relates also to the use of the package tampering and humidity sensitive sensor in packages and packaging material of foodstuff, electronics and perishable products, for monitoring whether the package or packaging material has been exposed to a humid or moist environment after activation of the sensor. The invention relates also to packages and packaging materials for foodstuff, electronics and perishable products comprising the package tampering and humidity sensitive sensor.

### Detailed Description of the Invention

It has now been surprisingly found that problems relating to package tampering and humidity sensors according to the state of the art can be avoided or at least significantly decreased by the package tampering and humidity sensitive sensor according to the present invention. The essential features of the invention are discussed as follows.

The sensor according to the present invention comprises a conductive polymer, such as polyaniline (PANI). It is a semiconductor in its emeraldine base form but it can be made conducting if it is protonated or doped with counter-ions, which bind to the iminic nitrogens of the polyaniline backbone. This process is typically a reversible reaction, i.e. the conductivity decreases when counter-ions are lost. The conductivity can be controlled using humidity sensitive counter-ions. In order to indicate reliably whether the sensor has been exposed to humid environment at any stage after activation it is essential that the reaction utilised is irreversible.

It has now been found that 5-formyl-2-furansulfonic acid (FFSA), as shown in the following formula I, is particularly suitable as the counter-ion of a conductive polymer, such as polyaniline, it acts as the dopant in the time and humidity sensor according to the invention, and provides an irreversible reaction when exposed to moisture.

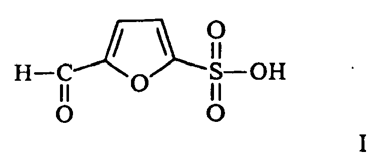

Hydrolysis of 5-formyl-2-furansulfonic acid takes place at humid conditions and may result in the decomposition of the whole molecule. When the hydrolysis reaction of 5-formyl-2-furansulfonic acid proceeds, also the conductivity of the conductive polymer, such as polyaniline, decreases because the decomposed 5-formyl-2-furansulfonic acid is not able to function as the dopant anymore.

### Structure of the sensor

The sensor according to the invention comprises a polymer layer comprising a conducting polymer and 5-formyl-2-furansulfonic acid, an optional substrate layer, an optional protective layer and optionally two electrodes.

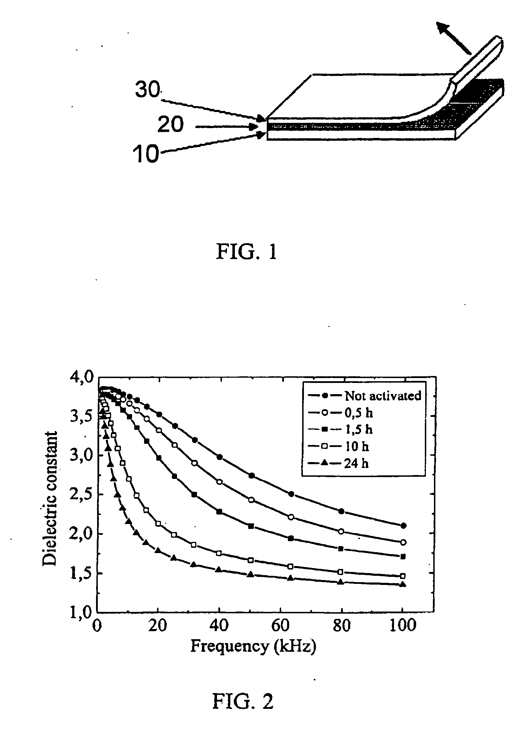

One preferable embodiment of invention is shown in Figure 1.

In Figure 1 the general structure of a sensor according to the invention is presented. It comprises a substrate 10, a polymer layer 20 of a mixture comprising a conducting polymer and 5-formyl-2-furansulfonic acid, and an optional protective layer 30. The optional protective layer is peeled off when the sensor is activated. The changes in the polymer layer, indicating changes in the humidity in the surroundings, are detected by electrical or optical measurements. The changes in the polymer layer, for example changes in resistivity, impedance or dielectric constant of the polymer layer (PANI layer) are examples of suitable electrical measurements but also all other electrical measurements are possible. As an optical characterization color chances can be detected by using visual means by eye, optical microscope, spectrometer or any methods, which detect changes in the optical spectrum.

In an alternative embodiment the sensor may comprise a polymer layer comprising a conducting polymer and 5-formyl-2-furansulfonic acid between two electrode layers, and an optional substrate layer under the first electrode layer and an optional protective layer on the second, outer electrode layer. In this embodiment the outer electrode layer is of humidity/moisture permeable material (like Indium thin film, disclosed in US 4,496,931 by Watanabe Masanori et al.) or material with small holes. In the case no substrate is used the first electrode layer, such as aluminum tape, may act as a substrate too. In this embodiment the indication takes place between the electrodes with capacitive or resistive measurements.

In a further alternative embodiment the sensor may comprise a polymer layer comprising a conducting polymer and 5-formyl-2-furansulfonic acid on two lateral electrodes on a substrate, and an optional protective layer. The lateral electrodes may be manufactured or glued or printed on the substrate layer. In this embodiment the indication takes place between the electrodes with optical, capacitive or resistive measurements.

In a further alternative embodiment the sensor may comprise a polymer layer comprising a conducting polymer and 5-formyl-2-furansulfonic acid and two interpenetrating fingerlike electrodes on or under the polymer layer, an optional substrate layer under the polymer layer and an optional protective layer on the top of the sensor. In this embodiment the indication takes place between the fingers with optical, capacitive or resistive measurements.

In a further alternative embodiment the sensor may comprise a polymer layer comprising a conducting polymer and 5-formyl-2-furansulfonic acid, which acts as the substrate layer too, and two electrodes adjacent with respect to each other, and an optional protective layer. In this embodiment the indication takes place using optical, capacitive or resistive measurements between the electrodes. In this case the sensor may also be in the form of pellets.

The substrate is selected from materials, which are sufficiently moisture-proof to protect the polymer layer from humidity. In some embodiments it is essential that the dielectric constant can be measured through the substrate. Suitably the substrate is selected from board, coated paperboard, paper, glass, plastic, metal foil, metallized film and waxed paper. Preferably the substrate is board or coated paperboard.

The polymer layer comprises a conductive polymer in protonated form, 5-formyl-2-furansulfonic acid, optional carriers and inert additives.

The conductive polymer is selected from polyaniline, polytiophene and polypyrrole and preferably polyaniline is used. The polymer layer comprises 9 - 84 % by weight, preferably 31 - 48 % by weight of the conductive polymer.

The polymer layer comprises 16-91 % by weight, preferably 52 - 69 % by weight of 5-formyl-2-furansulfonic acid as the dopant. The degree of doping varies between 0.1 and 5.0, preferably between 0.5 and 1.0.

Additionally the polymer layer may comprise 0.1 - 75 % by weight of additives and inert materials selected from polyvinylalcohol (PVA), polymethylmethacrylate (PMMA) and polystyrene.

The thickness of the polymer layer may vary between 10nm-5mm, preferably 50nm-500 nm.

When thin polymer layers, preferably 50 nm-500 nm are used the time and humidity sensor according to the invention is very responsive and the indication is fast.

The response may be adjusted as desired by varying the layer thickness. The response can be alternatively delayed when protective material is not peeled off and it is selected to have controlled and low enough water vapor (moisture) transmission rate.

The optional protective layer comprises a polymeric film, which is attached on the surface of the sensor completely or only from the edges in such manner that the polymer layer is not in direct contact with the surrounding environment and it can be same material as the substrate. The protective layer is preferably selected from an adhesive plastic film, adhesive tape or the like which can be easily torn off. The plastic material of the plastic film is selected from plastic materials with pre-determined humidity permeability. Particularly suitable are polyolefins, polyamides, polyvinylalcohols, blockcopolymers of polyurethanes and polyamides or of polyamides and polyethers.

The thickness of the protective layer may vary between 10nm-5mm, preferably 50nm-500µm.

The sensor according to the invention may comprise no protective layer, a tearable protective layer or a permanent protective layer, which has controlled and predetermined moisture transmission rate. In each of these cases the response rate may also be adjusted by altering the layer thickness of the sensing polymer layer or the protective layer.

The electrode may comprise any electrically conducting material having conductivity more than 100 fold of that of the polymer layer in the sensor according to the invention, preferably conducting filament of aluminum or copper tape, printing ink of silver, evaporated metals selected from Al, Au, Cu, Ag, Ni, carbon nanotubes or carbon paste, silver paste, stable conducting polymers selected from polyanilines, polythiophenes or polypyrroles.

The electrodes may comprise outer electrode layer and inner electrode layer, and the polymer layer is placed between the electrode layers, or the electrodes may comprise two lateral electrodes on a substrate and the polymer layer on the electrodes, or the electrodes may comprise two interpenetrating fingerlike electrodes on or under the polymer layer, or the electrodes may comprise two electrodes adjacent with respect to each other on the polymer layer.

### Method for the manufacture of the sensor

The method for the manufacture of the package tampering and humidity sensor according to the invention comprises the steps wherein a polymer mixture is formed by blending a conductive polymer in protonated form, 5-formyl-2-furansulfonic acid, optional inert additives and formic acid, and the obtained polymer mixture is formed to a layer or pellets. The polymer layer is manufactured from the polymer mixture by spraying, coating, printing or with a roll on a substrate or on an electrode or electrodes or between electrodes, optionally situated on a substrate. Preferable coating/printing methods are spraycoating, spincoating, dropcasting, gravure, flexo, screenprinting and inkjet.

The sensor according to the invention may also be in the form of pellets, or it may be incorporated into porous materials such as filters. The pellets are formed from the polymer mixture and electrode mixtures each containing two electrodes and an optional carrier and additives, suitably manufactured by pressing or extruding using equipment well known in the art.

The polymer mixture is formed by dissolving 0.1-50, preferably 0.5 - 2.0 % by weight of the conductive polymer in formic acid and 0.1-50, preferably 0.5 - 5.0 % by weight of 5-formyl-2-furansulfonic acid in formic acid, (preferably the concentration of formic acid is approx. 98%), combining the obtained solutions resulting in a solution comprising the ratio of the repeating unit of the conductive polymer to sulfonic acid of 0.1 -5.0, preferably 0.5 - 1.0, and then adding 0-75 % by weight of optional additives, carriers and solvents.

In the embodiments where a protective layer is used, the protective layer is attached on the polymer layer immediately after the applying of the polymer layer, using methods like spraying, laminating, gluing or coating.

In the embodiments where electrodes are used, the electrode layers or electrodes are manufactured by attaching with adhesives, as tapes, evaporating methods, printing and coating methods and the like on the substrate and/or on the polymer layer.

The package tampering and humidity sensor according to the invention comprising the protective layer is activated at a desired moment by tearing the protective layer off.

Alternatively, if the sensor is used for humidity detection inside packages, which should be kept tightly sealed or closed, and particularly for the detection whether the package has been opened or broken, no protection layer is needed. The sensor is manufactured on the package/package material, facing inside the package just before packaging of the goods or before filling with a protective gas atmosphere. The manufacturing time is also the moment of activation. Alternatively the sensor may comprise a protective layer, which is torn off at the time of closing the package.

Changes in the conductivity of the conductive polymer in the time and humidity sensor according to the invention, is suitably monitored by detecting or measuring a change in dielectric constant by using capacitance measurements where the sensor material acts as a conventional dielectric in device. Any chances in resistance or in impedance can be detected. Measuring frequency can be chosen to be at the most sensitive area. Accordingly the sensor may be manufactured as a changing resistor or an insulator and all suitable detection methods can be used.

Changes in the time and humidity sensor according to the invention may also be monitored optically by using known optical methods typically at wavelength of 500-800 nm. Depending on the wavelength the detection may be carried out visually or using UV-VIS or IR spectrofotometers.

The sensor according to the invention can be used for indicating if a package is opened or tampered and possibly how long time has passed since the opening event. In that case the opening of the package removes the protective layer and exposes the material to humidity.

Another application is to use the sensor in so-called low humidity packages (electronics, food supplies etc) to indicate electronically if the predetermined humidity limit is exceeded.

The package tampering and humidity sensor according to the invention has several advantages. It is easy to manufacture, it is cheap, easy to use i.e. to activate and to detect changes. It can be used in packages of any products, which are sensitive to humidity and moisture, such as perishable products, drugs, chemicals, food products, biological products and electronics etc. It can also be used as proof that packages are intact.

The following illustrative examples provide better understanding of the invention and its practical embodiments, however it is evident to a man skilled in the art that the scope of the invention is not limited to these examples in any way.

### EXAMPLES

### Example 1. Capacitive measurements

Capacitive measurements were performed with an AC LF-impedance analysator (HP 4192A) (a portable LCR/ESR-device can also be used) and with a special measuring platform having two lateral electrodes constructed for the purpose. Figure 1 shows the principled structure of the sensor and Figure 2 the measuring results of the dielectric constant of the sensor as a function of frequency when the sensor is not activated i.e. protected with adhesive plastic layer or after the sensor was activated and exposed to humidity (ca. 20 % RH) for 0,5 to 24 hours.

### Example 2. Effect of layer thickness on conductivity

The effect of film or layer thickness on the conductivity of a polymer layer containing 31.1 wt% of polyaniline and 68.9 wt% of FFSA, exposed to a humid environment (RH 50 %) for 7 days was studied using UV-Vis spectroscopy. The degree of doping and indirectly also the level of conductivity were revealed in the measurements. PANI(FFSA)_{1.0} having doping/blending ratio of 1.0, lost its conductivity and turned from green to blue when kept in a humidity chamber.

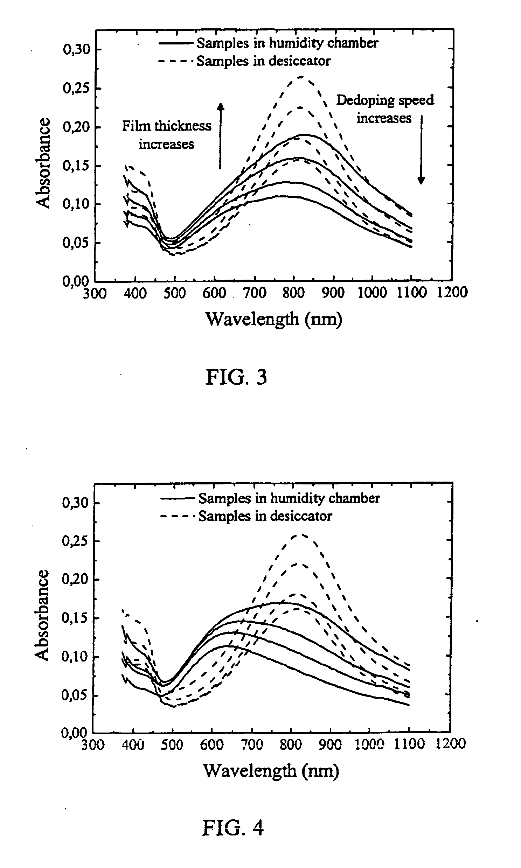

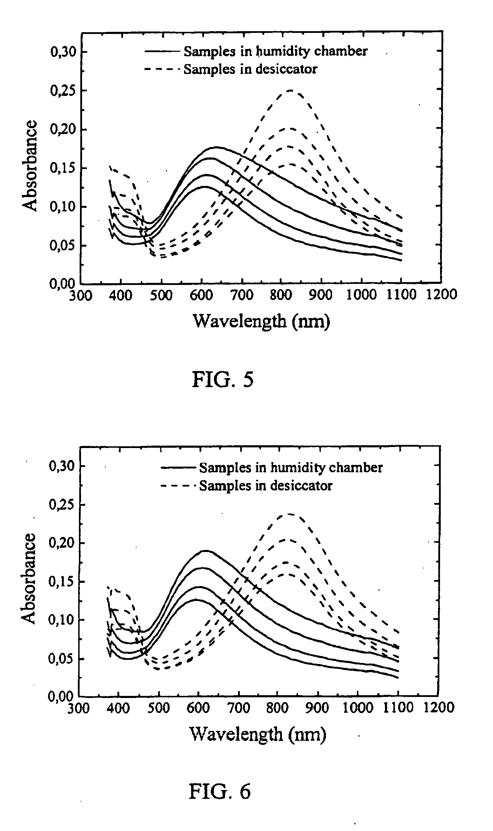

It was demonstrated in Figures 3-6 with UV-Vis spectroscopy that the hydrolysis rate of FFSA can be controlled with the film(layer) thickness. The changes in the conductivity can be measured either directly with resistance measurements or capacitively without touching the sample.

In the presence of humidity deprotonation or degradation of the polymer complex takes place in a PANI-FFSA layer. UV-Vis measurements clearly showed that deprotonation (dedoping) was the main reason behind the decrease in conductivity. This can be seen from the shift of the absorbance peak from 800 nm to 600 nm when time spent in the humidity chamber increases in Figures 3- 6. Completely doped polyaniline has a peak at 800 nm and dedoped polyaniline in the base form has a peak at 600 nm. A complex, which is partially doped, has a spectrum with both peaks, like shown in Figure 4. Reference samples were kept for the same time also in a desiccator having low humidity. The results are shown as dashed lines and they show that if no or only little moisture is present no changes are observed in the material. The measurement was performed for spin-cast films on a quartz glass slide. Casting speeds were 1000 (highest graph), 1500, 2500 and 3500 (lowest graph) rpm.

In Figures 3-6 the UV-Vis spectra of PANI(FFSA)_{1.0} = Pani EB 31.1 w-% and FFSA 68.9 w-%) for four different film thickness are presented (absorbance as a function of wavelength, different figures show the absorbance at different times 1h, 1 day, 4 days, 7 days). The data in the figures have not been shifted and thus the thinnest film is shown lowest and thickest film uppermost because thicker films absorb more light.

Samples were kept in a humidity chamber at 50 % relative humidity (RH) (solid line) or in a desiccator at low humidity (<10%) (dashed line) for 1 h (Figure 3), 1 day (Figure 4), 4 days (Figure 5) and 7 days (Figure 6). Additionally, the effect of humidity to the conductivity of the PANI(FFSA) films was followed at ambient conditions in the laboratory as a function of time.

### Example 3. Sheet resistance measurements

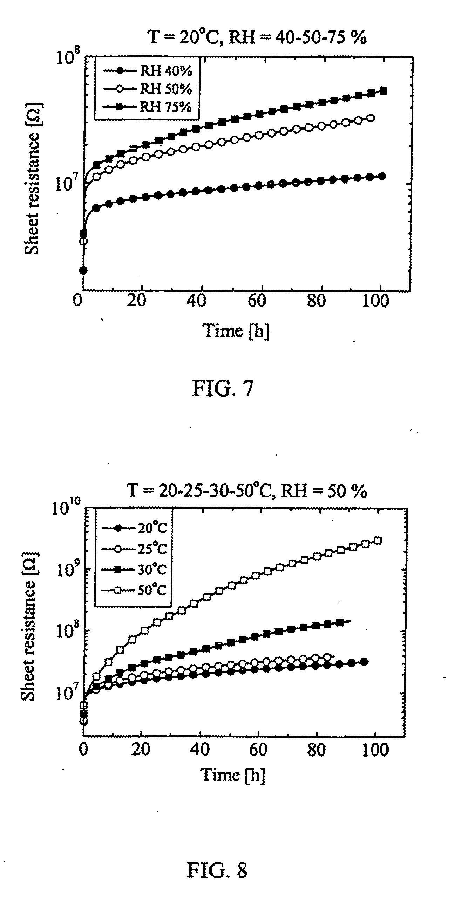

Sheet resistance measurements were carried out with four samples of PANI(FFSA)_{1.0} which was also used in the above examples. Temperature was first maintained constant at 20 °C and relative humidity was changed from 40 to 75 %. The results are presented in Fig. 7, where the relative humidity level is kept at 40 %, 50 % or 75 %.

In Fig. 8 the relative humidity was maintained constant 50 %, but the temperature was changed from 20 to 50 °C. The effect of humidity and temperature on sheet resistance can be seen from the figures, where temperature is kept at 20 °C, 25 °C, 30 °C or 50 °C.

## Claims

1. A package tampering and humidity sensor, **characterised in that** it comprises a polymer layer (20) comprising a conducting polymer and 5-formyl-2-furansulfonic acid, an optional substrate (10) layer, an optional protective layer (30) and optionally at least one electrode.

2. The package tampering and humidity sensor according to claim 1, **characterised in that** the substrate (10) is selected from board, coated paperboard, paper, glass, plastic, metal foil, metallized film and waxed paper, preferably the substrate (10) is board or coated paperboard.

3. The package tampering and humidity sensor according to claim 1 or 2, **characterised in that** the conductive polymer is selected from polyaniline, polythiophene and polypyrrole, preferably polyaniline.

4. The package tampering and humidity sensor according to any one of claims 1 - 3, **characterised in that** the polymer layer (20) comprises 9 - 84 % by weight, preferably 31 - 48 % by weight of the conductive polymer.

5. The package tampering and humidity sensor according to any one of claims 1 - 4, **characterised in that** the polymer layer (20) comprises 16 - 91 % by weight, preferably 52 - 69 % by weight of 5-formyl-2-furansulfonic acid.

6. The package tampering and humidity sensor according to any one of claims 1 - 5, **characterised in that** the polymer layer (20) comprises components selected from carriers and inert additives.

7. The package tampering and humidity sensor according to any one of claims 1 - 6, **characterised in that** the protective layer (30) comprises a polymeric film, which is attached on the surface of the sensor layer completely or only from the edges.

8. The package tampering and humidity sensor according to any one of claims 1 - 7, **characterised in that** the protective layer (30) is selected from adhesive plastic films and adhesive tapes.

9. The package tampering and humidity sensor according to any one of claims 1 - 8, **characterised in that** the electrode comprises an outer electrode layer and inner electrode layer and the polymer layer (20) is placed between the electrode layers, or the electrode comprises two lateral electrodes on the substrate (10) and the polymer layer (20) on the electrodes, or the electrode comprises two interpenetrating fingerlike electrodes on or under the polymer layer (20), or the electrode comprises two electrodes adjacent with respect to each other on the polymer layer (20).

10. A method for the manufacture of the package tampering and humidity sensor according to claim 1, **characterised in that** the method comprises the steps wherein a polymer mixture is formed by blending a conductive polymer in protonated form, 5-formyl-2-furansulfonic acid, optional carriers, inert additives, solvents and formic acid, and the obtained polymer mixture is formed to a layer or pellets.

11. The method according to claim 10, **characterised in that** the polymer mixture is formed to a layer by spraying, coating, printing or with a roll, on a substrate (10) or on an electrode or electrodes, optionally situated on a substrate (10), preferably by methods selected from spraycoating, spincoating, dropcasting, gravure, flexo, screenprinting and inkjet, or the polymer mixture is formed to pellets.

12. The method according to claim 10 or 11, **characterised in that** the polymer mixture is formed by dissolving 0.1-50 % by weight of the conductive polymer in formic acid and 0.1-50 % by weight of 5-formyl-2-furansulfonic acid in formic acid, combining the obtained solutions resulting in a solution comprising the ratio of the repeating unit of the conductive polymer to sulfonic acid of 0.1 -5.0, and then adding 0-75 % by weight of optional additives, carriers and solvents.

13. The method according to claim 12, **characterised in that** the polymer mixture is formed by dissolving 0.5 - 2.0 % by weight of the conductive polymer in formic acid and 0.5 - 5.0 % by weight of 5-formyl-2-furansulfonic acid in formic acid, combining the obtained solutions resulting in a solution comprising the ratio of the repeating unit of the conductive polymer to sulfonic acid of 0.5 - 1.0.

14. The method according to any one of claims 10-13, **characterised in that** a protective layer (30) is attached on the polymer layer (20) using a method selected from spraying, laminating, gluing and coating.

15. The method according to any one of claims 10-14, **characterised in that** electrode layers or electrodes are manufactured by attaching with adhesives, as tapes, evaporating methods, printing and coating methods and the like on the substrate (10) and/or on the polymer layer (20).

16. Use of the package tampering and humidity sensor according to any one of claims 1- 9 in packages and packaging materials of goods.

17. Use according to claim 16, **characterised in that** the goods are electronics, food products, biological materials and perishable products.

## Patentansprüche

1. Manipulations- und Feuchtigkeitsmeßfühler einer Verpackung, **dadurch gekennzeichnet, dass** der Fühler eine ein leitfähiges Polymer und 5-Formyl-2-furansulfonsäure enthaltende Polymerschicht (2), gegebenenfalls eine Substratschicht (10), gegebenenfalls eine Schutzschicht (30) und gegebenenfalls mindestens eine Elektrode aufweist.

2. Manipulations- und Feuchtigkeitsmeßfühler einer Verpackung nach Anspruch 1, **dadurch gekennzeichnet, dass** das Substrat aus der Gruppe, die aus Karton, beschichteter Pappe, Papier, Glas, Kunststoff, Metallfolie, metallisierter Folie und gewachstem Papier besteht, ausgewählt ist, wobei das Substrat (10) vorzugsweise aus Karton oder beschichteter Pappe besteht.

3. Manipulations- und Feuchtigkeitsmeßfühler einer Verpackung nach Anspruch 1 oder 2, **dadurch gekennzeichnet, dass** das leitfähige Polymer aus der Gruppe, die aus Polyanilin, Polythiophen and Polypyrrol besteht, ausgewählt ist, bevorzugt Polyanilin ist.

4. Manipulations- und Feuchtigkeitsmeßfühler einer Verpackung nach einem der Ansprüche 1-3, **dadurch gekennzeichnet, dass** die Polymerschicht (20) von 9 bis 84 Gew.%, bevorzugt von 31 bis 48 Gew.% des leitfähigen Polymers enthält.

5. Manipulations- und Feuchtigkeitsmeßfühler einer Verpackung nach einem der Ansprüche 1-4, **dadurch gekennzeichnet, dass** die Polymerschicht (20) von 16 bis 91 Gew.%, bevorzugt von 52 bis 69 Gew.% der 5-Formyl-2-furansulfonsäure enthält.

6. Manipulations- und Feuchtigkeitsmeßfühler einer Verpackung nach einem der Ansprüche 1-5, **dadurch gekennzeichnet, dass** die Polymerschicht (20) aus der Gruppe von Trägern und inerten Zusatzmitteln ausgewählte Komponente enthält.

7. Manipulations- und Feuchtigkeitsmeßfühler einer Verpackung nach einem der Ansprüche 1-6, **dadurch gekennzeichnet, dass** die Schutzschicht (30) eine Polymerfolie aufweist, die entweder vollflächig oder nur an den Rändern an der Oberfläche der Meßfühlerschicht befestigt ist

8. Manipulations- und Feuchtigkeitsmeßfühler einer Verpackung nach einem der Ansprüche 1-7, **dadurch gekennzeichnet, dass** die Schutzschicht (30) aus der Gruppe der Klebfolien aus Kunststoff und Klebebänder ausgewählt ist.

9. Manipulations- und Feuchtigkeitsmeßfühler einer Verpackung nach einem der Ansprüche 1-8, **dadurch gekennzeichnet, dass** die Elektrode eine äußere Elektrodenschicht und eine innere Elektrodenschicht aufweist und die Polymerschicht (20) zwischen den Elektrodenschichten angeordnet ist, oder die Elektrode zwei auf dem Substrat (10) angeordneten Seitenelektroden aufweist und die Polymerschicht (20) auf den Elektroden angeordnet ist, oder die Elektrode zwei ineinander eindringende fingerartige Elektroden auf oder unter der Polymerschicht (20) aufweist, oder die Elektrode zwei auf der Polymerschicht (20) nebeneinander angeordneten Elektroden aufweist.

10. Verfahren zur Herstellung eines Manipulations- und Feuchtigkeitsmeßfühlers einer Verpackung nach Anspruch 1, **dadurch gekennzeichnet, dass** das Verfahren die folgenden Schritte umfaßt, in denen ein Polymergemisch durch Mischen eines leitfähigen Polymers in der protonierten Form mit 5-Formyl-2-furansulfonsäure, gegebenenfalls mit Trägern, inerten Zusatzmitteln, Lösungsmitteln und Ameisensäure hergestellt und die erhaltene Polymergemisch in eine Schicht oder Pellets umgewandelt wird.

11. Verfahren nach Anspruch 10, **dadurch gekennzeichnet, dass** das Polymergemisch auf einem Substrat (10) oder auf einer gegebenenfalls auf dem Substrat (10) angeordneten Elektrode oder Elektroden in eine Schicht durch Spritzen, Beschichten, Drucken oder mit einer Walze umgewandelt wird, bevorzugt durch Verfahren, die aus Spritzbeschichtung, Schleuderbeschichtung, Tropfgießen, Tiefdruck, Flexodruck, Siebdruck und Tintenstrahldruck ausgewählt sind, oder das Polymergemisch zu Pellets umgewandelt wird.

12. Verfahren nach Anspruch 10 oder 11, **dadurch gekennzeichnet, dass** das Polymergemisch durch getrenntes Auflösen von 0,1-50 Gew.% des leitfähigen Polymers und 0,1-50 Gew.% der 5-Formyl-2-furansulfonsäure jeweils in Ameisensäure, Kombinieren der erhaltenen Lösungen miteinander zu einer Lösung, in der das Verhältnis der Bausteine des leitfähigen Polymerz zu Sulfonsäure von 0,1 bis 5,0 beträgt, und danach gegebenenfalls durch Zusatz von 0-75 Gew.% der Zusatzmittel, Träger und Lösungsmittel hergestellt wird.

13. Verfahren nach Anspruch 12, **dadurch gekennzeichnet, dass** das Polymergemisch durch getrenntes Auflösen von 0,5-2,0 Gew.% des leitfähigen Polymers und 0,5-5,0 Gew.% der 5-Formyl-2-furansulfonsäure jeweils in Ameisensäure, Kombinieren der erhaltenen Lösungen miteinander zu einer Lösung, in der das Verhältnis der Bausteine des leitfähigen Polymerz zu Sulfonsäure von 0,5 bis 1,0 beträgt,

14. Verfahren nach einem der Ansprüche 10-13, **dadurch gekennzeichnet, dass** die Schutzschicht (20) an der Polymerschicht (20) durch ein unter Spritzen, Laminieren, Kleben und Beschichten ausgewähltes Verfahren befestigt ist.

15. Verfahren nach einem der Ansprüche 10-15, **dadurch gekennzeichnet, dass** die Elektrodenschichten oder Elektroden durch Kleben, als Klebebänder, mit Bedampfungs-, Druck-, Beschichtungs- und ähnlichen Verfahren auf dem Substrat (10) und/oder auf der Polymerschicht (20) hergestellt sind.

16. Verwendung des Manipulations- und Feuchtigkeitsmeßfühlers einer Verpackung nach einem der Ansprüche 1-9 in Verpackungen und Verpackungsmaterialien für Güter.

17. Verwendung nach Anspruch 16, **dadurch gekennzeichnet, dass** die Güter elektronische Produkte, Lebensmittel, biologische Materialien und verderbliche Produkte sind.

## Revendications

1. Capteur de manipulation et humidité pour un emballage, **caracterisé en ce qu**'il comprend une couche de polymère (20) comprenant un polymère conducteur et l'acide formyle 5-furannesulfonique, éventuellement une couche de substrat (10), éventuellement une couche de protection (30) et éventuellement au moins une électrode.

2. Capteur de manipulation et humidité pour un emballage selon la revendication 1, **caracterisé en ce que** le substrat est choisi parmi carton, carton enduit, papier, verre, matière plastique, feuille de métal, feuille métallisée et papier ciré, et préférentiellement le substrat (10) est carton ou carton enduit.

3. Capteur de manipulation et humidité pour un emballage selon la revendication 1 ou 2, **caracterisé en ce que** le polymère conducteur est choisi parmi polyaniline, polythiophène et polypyrrole, préférentiellement polyaniline.

4. Capteur de manipulation et humidité pour un emballage selon une des revendications 1-3, **caracterisé en ce que** la couche de polymère (20) comprend 9 à 82 %, préférentiellement 31 à 48 % du polymère conducteur, en poids.

5. Capteur de manipulation et humidité pour un emballage selon une des revendications 1-4, **caracterisé en ce que** la couche de polymère (20) comprend 16 à 91 %, préférentiellement 52 à 69 % de l'acide formyle 5-furannesulfonique, en poids.

6. Capteur de manipulation et humidité pour un emballage selon une des revendications 1-5, **caracterisé en ce que** la couche de polymère (20) comprend des composants choisi parmi des supports et additifs inertes.

7. Capteur de manipulation et humidité pour un emballage selon une des revendications 1-6, **caracterisé en ce que** la couche de protection (20) comprend un film de polymère attaché totalement ou seulement par des bords á la surface de la couche de capteur.

8. Capteur de manipulation et humidité pour un emballage selon une des revendications 1-7, **caracterisé en ce que** la couche de protection (30) est choisie parmi des films plastiques adhésives et bandes adhésives.

9. Capteur de manipulation et humidité pour un emballage selon une des revendications 1-8, **caracterisé en ce que** l'électrode comprend une couche extérieur d'électrode et une couche intérieur d'électrode et la couche de polymère (20) est placèe entre ces couches d'électrodes, ou l'électrode comprend deux électrodes laterales sur le substrat (10) et la couche de polymère (20) sur les électrodes, ou l'électrode comprend deux électrodes du type doigt imbriqués sur ou sous la couche de polymère (20), ou l'électrode comprend deux électrodes adjacentes sur la couche de polymère (20).

10. Procédé de preparation d'un capteur de manipulation et humidité pour un emballage selon la revendication 1, **caracterisé en ce que** le procédé comprend les étapes suivantes: formation du mélange polymère par mélange le polymère conducteur en forme protonée, l'acide formyle 5-furannesulfonique, éventuellement des supports, additifs inertes, solvants et l'acide formique, et formation d'une couche ou pellets de cet mélange polymère obtenu.

11. Procédé selon la revendication 10, **caracterisé en ce que** le mélange polymère est transformé en une couche par atomisation, enduction, impression ou avec un cylindre sur le substrat (10) ou sur l'électrode ou les électrodes, éventuellement placée(s) sur le substrat (10), préférentiellement par méthodes choisies parmi enduction par atomisation, enduction centrifuge, fonte egouttage, impression de gravure, de flexo, de screen, et impression jet d'encre, ou le mélange polymère est transformé en pellets.

12. Procédé selon la revendication 10 ou 11, **caracterisé en ce que** le mélange polymère est formé par dissolution séparée de 0,1-50 %, en poids, du polymère conducteur et de 0,1-50 %, en poids, de l'acide formyle 5-furannesulfonique respectivement dans l'acide formique, réunion des solutions formées pour obtenir une solution, dans laquelle le rapport des unités répétées du polymère conducteur à l'acide sulphonique est entre 0,1 et 5,0, éventuellement suivié par l'addition de 0-75 %, en poids, des additifs, supports et solvants.

13. Procédé selon la revendication 12, **caracterisé en ce que** le mélange polymère est formé par dissolution séparée de 0,5-2,0 %, en poids, du polymère conducteur et de 0,5-5,0 %, en poids, de l'acide formyle 5-furannesulfonique respectivement dans l'acide formique, réunion des solutions formées pour obtenir une solution, dans laquelle le rapport des unités répétées du polymère conducteur à l'acide sulphonique est entre 0,5 et 1,0.

14. Procédé selon une des revendications 10-13, **caracterisé en ce que** la couche de protection (30) est attachée à la couche polymère (20) par une méthode choisie parmi atomisation, lamination, collage et enduction.

15. Procédé selon une des revendications 10-13, caracterisé en ce des couches électrodes, ou électrodes sont préparées par collage, en tant que bandes adhésives, par méthodes d'évaporation, d'impression et d'enduction, et méthodes similaires, sur le substrat (10) et/ou sur la couche polymère (20).

16. Utilisation d'un capteur de manipulation et humidité pour un emballage selon une des revendications 1-9 dans les emballages et matériaux d'emballage pour marchandises.

17. Utilisation selon la revendication 16, **caracterisé en ce que** les marchandises sont des produits electroniques, aliméntaires ou périssables et des matériaux biologiques.
